# EUROPÄISCHE PATENTSCHRIFT

(11) **EP 1 379 486 B1**
(45) Veröffentlichungstag und Bekanntmachung des Hinweises auf die Patenterteilung: **09.06.2010**
(21) Anmeldenummer: 02766623.9
(22) Anmeldetag: 10.04.2002
(51) Int. Cl.: C07C 7/00, C08F 6/00

(54) **VERFAHREN ZUR AUFARBEITUNG EINES FLÜSSIGEN REAKTIONSAUSTRAGES DER KATIONISCHEN POLYMERISATION VON ISOBUTEN**
METHOD FOR THE PROCESSING OF A LIQUID REACTION DISCHARGE OF THE CATIONIC POLYMERISATION OF ISOBUTENE
PROCEDE POUR RETRAITER UN RESIDU DE REACTION LIQUIDE ISSU DE LA POLYMERISATION CATIONIQUE D'ISOBUTENE

(30) Priorität: 11.04.2001 DE 10118182
(43) Veröffentlichungstag der Anmeldung: 14.01.2004
(73) Patentinhaber: BASF SE, 67056 Ludwigshafen (DE)
(72) Erfinder: WETTLING, Thomas, 67117 Limburgerhof (DE); BORCHERS, Dirk, B-2950 Kapellen (BE); AUER, Heinz, 68809 Neulussheim (DE)
(86) Internationale Anmeldenummer: PCT/EP2002/004000
(87) Internationale Veröffentlichungsnummer: WO 2002/088053

(56) Entgegenhaltungen:
- EP-A- 0 435 116
- EP-A- 1 026 175
- WO-A-99/60028
- US-A- 3 280 091

## Beschreibung

Die vorliegende Erfindung betrifft ein Verfahren zur Aufarbeitung eines flüssigen Reaktionsaustrages der kationischen Polymerisation von Isobuten, der im Wesentlichen aus Polyisobuten, nicht umgesetztem Isobuten und einem inerten Verdünnungsmittel besteht.

Hochmolekulare Polyisobutene mit Molekulargewichten bis zu mehreren 100000 Dalton sind seit langem bekannt und ihre Herstellung ist beispielsweise in H. Güterbock: Polyisobutylen und Mischpolymerisate, S. 77-104, Springer, Berlin 1959, beschrieben. Von diesen herkömmlichen Polyisobutenen sind die sogenannten hochreaktiven Polyisobutene zu unterscheiden, welche einen hohen Gehalt an endständigen Vinylidengruppierungen von vorzugsweise deutlich über 60 Mol-% haben. Hochreaktive Polyisobutene sind als Zwischenprodukte zur Herstellung von Additiven für Schmier- und Kraftstoffe begehrt.

Derartige hochreaktive Polyisobutene sind z. B. nach dem Verfahren der EP 0 628 575 durch kationische Polymerisation von Isobuten in flüssiger Phase mit Hilfe von Bortrifluorid und eines sekundären Alkohols bei Temperaturen von 0 °C bis -60 °C erhältlich.

Die älteren Patentanmeldungen DE 199 48 947.5, DE 199 52 031.3, DE 199 52 030.5, DE 100 28 585.6 und DE 100 35 298.7 betreffen Verbesserungen oder vorteilhafte Ausgestaltungen eines derartigen Verfahrens.

Aufgrund der bei Raumtemperatur und darunter vergleichsweise hohen Viskosität von Polyisobuten wird die Polymerisation des Isobutens in der Regel in Gegenwart eines inerten Verdünnungsmittels durchgeführt. Das verwendete Verdünnungsmittel dient vor allem dazu, die während der Polymerisationsreaktion zu beobachtende Erhöhung der Viskosität des Reaktionsgemisches soweit zu verringern, dass die entstehende Reaktionswärme ausreichend rasch abgeführt werden kann. Nach Erreichen des gewünschten Molekulargewichts wird die Reaktion durch Deaktivierung des Polymerisationskatalysators abgebrochen. Vorzugsweise wird der Polymerisationskatalysator oder dessen Deaktivierungsprodukte durch Extraktion mit einer wässrigen Lösung entfernt.

Anschließend wird das gebildete Polyisobuten von dem nicht umgesetzten Isobuten und dem Verdünnungsmittel befreit, was üblicherweise durch Destillation erfolgt, wobei zuerst das leichtflüchtige Isobuten und anschließend das gegenüber Isobuten höhersiedende Verdünnungsmittel abdestilliert wird. Das Polyisobuten verbleibt als Destillationsrückstand. Die thermische Belastung bei der Destillation ist jedoch problematisch, weil Reste von Deaktivierungsprodukten des Polymerisationskatalysators, wie zum Beispiel Fluorwasserstoffspuren, beim Erwärmen zu unerwünschten Nebenreaktionen des nicht umgesetzten Isobutens oder Folgereaktionen des gebildeten Polyisobutens führen können. Eine unerwünschte Folgereaktion ist zum Beispiel die sauer katalysierte Isomerisierung eines Polyisobutenmoleküls mit endständiger Vinylidendoppelbindung zu einem Polyisobutenmolekül, bei dem die Doppelbindung eine thermodynamisch günstigere Position im Inneren des Moleküls einnimmt. Unerwünschte Nebenreaktionen des nicht umgesetzten Isobutens sind beispielsweise die Dimerisierung zu Isookten oder die Bildung von tert-Butanol aus Isobuten und im Reaktionsgemisch enthaltenen Wasserspuren.

Grundsätzlich ist man bestrebt, die für eine Destillation eingesetzte Heiz- und Kühlenergie möglichst wirtschaftlich zu verwenden und ein Aufheizen auf ein sehr hohes oder Abkühlen auf ein sehr tiefes Temperaturniveau zu vermeiden.

Der vorliegenden Erfindung liegt daher die Aufgabe zugrunde, ein Verfahren zur Aufarbeitung eines flüssigen Reaktionsaustrages der kationischen Polymerisation von Isobuten anzugeben, das unerwünschte Neben- und Folgereaktionen weitgehend vermeidet und einen effizienten Energieeinsatz gestattet.

Erfindungsgemäß wird diese Aufgabe durch ein Verfahren gelöst, bei dem man den Reaktionsaustrag erwärmt und in einen Entspannungsbehälter entspannt, wobei sich der Reaktionsaustrag infolge der Entspannung in eine das Polyisobuten enthaltende Flüssigphase und eine die Hauptmenge des im Reaktionsaustrag enthaltenen Isobutens und wenigstens 30 % des im Reaktionsaustrag enthaltenen Verdünnungsmittels enthaltende Gasphase auftrennt.

Der Reaktionsaustrag enthält typischerweise 20 bis 60 Gew.-% Polyisobuten, 0,5 bis 20 Gew.-% Isobuten und 40 bis 79,5 Gew.-% Verdünnungsmittel. Er kann daneben untergeordnete Mengen anderer Bestandteile, wie Spuren des zugesetzten Katalysatordeaktivierungsmittels, z. B. Alkohol, oder Folgeprodukte davon mit Isobuten, insbesondere Ether, Spuren von Fluorwasserstoff oder Wasser enthalten. Diese Bestandteile sind im Allgemeinen in einer Menge von weniger als 5 Gew.-%, meist weniger als 1 Gew.-%, vorhanden.

Der Reaktionsaustrag, in dem der Polymerisationskatalysator deaktiviert worden ist und/oder aus dem der Polymerisationskatalysator oder Deaktivierungsprodukte davon vorzugsweise entfernt worden sind, wie dies im Einzelnen weiter unten beschrieben ist, wird, beispielsweise in einem Wärmetauscher, vorzugsweise auf eine Temperatur von 40 bis 200 °C, insbesondere 40 bis 140 °C, besonders bevorzugt 40 bis 120 °C erwärmt. Der Reaktionsaustrag steht dabei unter einem Druck, der in der Regel dem des Polymerisationssystems entspricht und zum Beispiel 2 bis 30 bar, vorzugsweise 2 bis 20 bar beträgt. Anschließend wird der erwärmte Reaktionsaustrag in einen Entspannungsbehälter entspannt, der unter einem Druck von im Allgemeinen 1 bis 10 bar, vorzugsweise 1 bis 8 bar, besonders bevorzugt 1 bis 5 bar steht. Die Druckdifferenz der Entspannung beträgt vorzugsweise wenigstens 1 bar, insbesondere wenigstens 3 bar.

Infolge der Entspannung trennt sich der Reaktionsaustrag in eine das Polyisobuten enthaltende Flüssigphase und eine die Hauptmenge des im Reaktionsaustrages enthaltenen Isobutens und die Hauptmenge des im Reaktionsaustrag enthaltenen Verdünnungsmittels enthaltende Gasphase auf. Vorzugsweise wird die Temperatur, auf die der Reaktionsaustrag vorerwärmt wird, und die Druckdifferenz der Entspannung so gewählt, dass die Gasphase wenigstens 90 %, insbesondere wenigstens 95 %, des im Reaktionsaustrag enthaltenen Isobutens und wenigstens 30 %, insbesondere wenigstens 40 %, besonders bevorzugt wenigstens 60 %, des im Reaktionsaustrag enthaltenen Verdünnungsmittels enthält. Geeignete Kombinationen von Temperatur und Druckdifferenz kann der Fachmann aufgrund der bekannten Werte der spezifischen Wärmekapazität von Polyisobuten, Isobuten und des Verdünnungsmittels sowie der spezifischen Verdampfungsenthalpie von Isobuten und des Verdünnungsmittels ohne weiteres abschätzen oder alternativ durch einfache Versuche ermitteln.

Die bei der Entspannung entstehende Gasphase wird abgezogen und zweckmäßigerweise zu einem flüssigen Isobuten-Verdünnungsmittel-Gemisch kondensiert, das - zweckmäßigerweise nach einer Wäsche mit Wasser - in die Polymerisationsreaktion zurückgeführt werden kann. Vorteilhafterweise kondensiert das Isobuten-Verdünnungsmittel-Gemisch bereits bei höherer Temperatur als reines Isobuten, da die Verflüssigung des Isobutens durch dessen physikalische Löslichkeit in dem Verdünnungsmittel begünstigt wird. Da damit das Erfordernis entfällt, zur Kondensation des Isobutens auf sehr tiefe Temperaturen abzukühlen, ist das erfindungsgemäße Verfahren sehr wirtschaftlich.

Die bei der Entspannung anfallende Flüssigphase wird von gegebenenfalls vorhandenen Resten an nicht umgesetzten Isobuten und Verdünnungsmittel befreit, zum Beispiel durch einfache Destillation oder erneute Erwärmung und Entspannung.

Die Gestalt des Entspannungsbehälters unterliegt keinen wesentlichen Beschränkungen. Vorzugsweise wird der erwärmte Reaktionsaustrag so in den Entspannungsbehälter eingeführt, dass beim Eintritt in den Behälter eine große Flüssigkeitsoberfläche erzeugt wird, die das Ausdampfen der Gasphase aus der Flüssigphase begünstigt. Hierzu hat es sich als vorteilhaft erwiesen, einen vorzugsweise länglichen, vertikal angeordneten Entspannungsbehälter mit kreisförmigem Querschnitt zu verwenden und den erwärmten Reaktionsaustrag tangential zur Wand des Entspannungsbehälters, vorzugsweise in einer Richtung senkrecht zur Längsachse des Entspannungsbehälters, einzuführen. Auf diese Weise strömt der Reaktionsaustrag entlang der Behälterwand und folgt deren Krümmung, wodurch der Reaktionsaustrag einen Drall erfährt und sich im Entspannungsbehälter in Spirallinien entlang des Umfangs der Behälterwand nach unten bewegt.

Bevorzugt verwendet man als Entspannungsbehälter eine Kolonne, wobei die Zufuhr des erwärmten Reaktionsaustrages seitlich in einen von Einbauten freien Bereich der Kolonne, vorzugsweise im Bereich der Kolonnenmitte oder im oberen Bereich der Kolonne, erfolgt. Geeigneterweise leitet man die sich bildende Flüssigphase über trennwirksame Einbauten im unteren Bereich der als Entspannungsbehälter verwendeten Kolonne, um eine möglichst große Phasengrenzfläche zu schaffen und das Ausdampfen der Gasphase zu vervollständigen. Als trennwirksame Einbauten zum Beispiel sind Glockenböden oder bevorzugt eine Füllkörperpackung geeignet. Eine Sumpfheizung kann angewendet werden, diese ist aber für das erfindungsgemäße Verfahren nicht wesentlich.

Bei dem erfindungsgemäßen Verfahren wird das im Reaktionsaustrag enthaltene Polyisobuten nur kurzzeitig thermisch belastet, so dass Folgereaktionen, wie unerwünschte Isomerisierungen, zurückgedrängt werden. Dabei ist es zusätzlich von Vorteil, dass die im Reaktionsaustrag enthaltenen Wasserspuren bei der erfindungsgemäßen Entspannung weitgehend in die Gasphase übertreten.

Die kationische Polymerisation von Isobuten in Gegenwart eines Lewis-Säure-Katalysators kann kontinuierlich oder diskontinuierlich erfolgen, erfolgt jedoch vorzugsweise kontinuierlich. Verfahren zur kontinuierlichen Polymerisation in flüssiger organischer Phase sind an sich bekannt. Bei einem kontinuierlichen Verfahren wird kontinuierlich ein Teil der im Polymerisationsreaktor entstandenen Reaktionsmischung ausgetragen. Eine dem Austrag entsprechende Menge an Einsatzmaterialien, hier Isobuten bzw. Isobuten-haltiger Zulauf, wird dem Polymerisationsreaktor kontinuierlich zugeführt. Das Verhältnis von der im Polymerisationsreaktor befindlichen Stoffmenge zu der Menge, die ausgetragen wird, bestimmt sich durch das Umlauf/Zulauf-Verhältnis, das bei der kontinuierlichen Polymerisation von Isobuten zu Polyisobuten in der Regel im Bereich von 1000:1 bis 1:1, bevorzugt im Bereich von 500:1 bis 5:1 und insbesondere im Bereich von 200:1 bis 30:1 liegt. Die mittlere Verweildauer des zu polymerisierenden Isobutens im Polymerisationsreaktor kann fünf Sekunden bis mehrere Stunden betragen. Verweilzeiten von 1 bis 30 Minuten, insbesondere 2 bis 20 Minuten, sind besonders bevorzugt.

Die Polymerisation des Isobutens erfolgt in den üblichen Reaktoren, wie Rührkessel, Rohr-, Rohrbündel- und Schlaufenreaktoren, wobei Schlaufenreaktoren, d. h. Rohr(bündel)reaktoren mit Rührkesselcharakteristik, bevorzugt sind. Besonders günstig sind Rohrreaktoren mit Rohrquerschnitten, die in Teilbereichen zu Turbulenzen führen.

Die Polymerisation wird üblicherweise bei einer Reaktionstemperatur von -60 bis +40 °C, insbesondere -30 bis 0 °C, besonders bevorzugt -25 bis -5 °C, durchgeführt. Die Polymerisationswärme wird entsprechend mit Hilfe einer Kühlvorrichtung abgeführt. Diese kann beispielsweise mit flüssigem Ammoniak als Kühlmittel betrieben werden. Eine andere Möglichkeit, die Polymerisationswärme abzuführen, ist die Siedekühlung. Dabei wird die freiwerdende Wärme durch teilweises Verdampfen des Reaktionsgemischs, z. B. des Isobutens und/oder anderer leicht flüchtiger Bestandteile des Isobutenzulaufs oder eines leicht flüchtigen Verdünnungsmittels, abgeführt. Vorzugsweise arbeitet man unter isothermen Bedingungen, d. h. die Temperatur der flüssigen organischen Reaktionsphase im Polymerisationsreaktor hat einen stationären Wert und ändert sich während des Betriebs des Reaktors nicht oder nur in geringem Maße.

Die Konzentration des Isobutens in der flüssigen Reaktionsphase liegt in der Regel im Bereich von 0,2 bis 50 Gew.-%, vorzugsweise im Bereich von 0,5 bis 20 Gew.-%, bezogen auf die flüssige organische Phase.

Als Einsatzstoffe eignen sich sowohl Isobuten selbst als auch Isobuten-haltige C₄-Kohlenwasserstoffströme, beispielsweise C₄-Raffinate, C₄-Schnitte aus der Isobutan-Dehydrierung, C₄-Schnitte aus Steamcrackern, FCC-Crackern (fluid catalysed cracking), sofern sie weitgehend von darin enthaltenem 1,3-Butadien befreit sind. Geeignete C₄-Kohlenwasserstoffströme enthalten in der Regel weniger als 500 ppm, vorzugsweise weniger als 200 ppm, Butadien. Die Anwesenheit von 1-Buten, cis- und trans-2-Buten ist weitgehend unkritisch. Typischerweise liegt die Isobutenkonzentration in den C₄-Kohlenwasserstoffströmen im Bereich von 40 bis 60 Gew.-%. Bei Einsatz von C₄-Schnitten als Einsatzmaterial übernehmen die von Isobuten verschiedenen Kohlenwasserstoffe die Rolle eines inerten Verdünnungsmittels. Der Isobuten-haltige Zulauf kann geringe Mengen an Kontaminanten, wie Wasser, Carbonsäuren oder Mineralsäuren enthalten, ohne dass es zu kritischen Ausbeute- oder Selektivitätseinbußen kommt. Es ist zweckdienlich, eine Anreicherung dieser Verunreinigungen zu vermeiden, indem man solche Schadstoffe beispielsweise durch Adsorption an feste Adsorbentien, wie Aktivkohle, Molekularsiebe oder Ionenaustauscher, aus dem Isobuten-haltigen Zulauf entfernt.

Als Verdünnungsmittel sind solche Lösungsmittel oder Lösungsmittelgemische geeignet, die gegenüber den eingesetzten Reagenzien inert sind. Geeignete Verdünnungsmittel sind beispielsweise gesättigte Kohlenwasserstoffe, wie Butan, Pentan, Hexan, Heptan, Octan, z. B. n-Hexan, i-Octan, Cyclopentan, halogenierte Kohlenwasserstoffe, wie Methylchlorid, Dichlormethan oder Trichlormethan, sowie Mischungen der vorgenannten Verdünnungsmittel, wovon n-Hexan besonders bevorzugt ist. Vorzugsweise werden die Verdünnungsmittel vor ihrem Einsatz von Verunreinigungen wie Wasser, Carbonsäuren oder Mineralsäuren befreit, beispielsweise durch Adsorption an feste Adsorbentien, wie Aktivkohle, Molekularsiebe oder Ionenaustauscher.

Es ist für die Abführung der Reaktionswärme günstig, bei großer Verdünnung, d. h. mit einem hohen Verdünnungsmittelanteil im Reaktionsgemisch zu arbeiten. Andererseits verringert ein hoher Verdünnungsmittelanteil die pro Reaktorvolumen erzielbare Menge an Polyisobuten und verschlechtert die Wirtschaftlichkeit des Verfahrens. In der Praxis wird man daher einen Kompromiss zwischen hoher Reaktorvolumenausnutzung und - für die Abführung der Reaktionswärme - ausreichend geringer Viskosität bei Reaktionstemperatur wählen. Die optimale Verdünnungsmittelmenge kann vom Fachmann in einfacher Weise bestimmt werden, indem er den Verdünnungsmittelanteil im Reaktionsgemisch bis knapp vor den Punkt vermindert, an dem die Reaktionswärme nicht mehr rasch genug abgeführt werden kann. Das Unterschreiten der optimalen Verdünnungsmittelmenge ist an einem Temperaturanstieg im Reaktor und gegebenenfalls einer beginnenden Verschlechterung der Produktqualität erkennbar.

Als Lewis-Säure-Katalysator ist Bortrifluorid, vorzugsweise in Kombination mit einem Cokatalysator, besonders bevorzugt. Bortrifluorid wird zweckmäßigerweise in Form von gasförmigem Bortrifluorid eingesetzt, wobei technisches, noch geringe Mengen Schwefeldioxid und SiF₄ enthaltendes, vorzugsweise aber hochreines Bortrifluorid mit einer Reinheit von etwa 99,5 Gew.-% verwendet werden kann.

Geeignete Cokatalysatoren sind in der Regel sauerstoffhaltige Verbindungen, die vorzugsweise wenigstens ein zweibindiges Sauerstoffatom enthalten. Geeignete sauerstoffhaltige Verbindungen sind neben Wasser organische Verbindungen bis zu 30 Kohlenstoffatomen. Beispiele hierfür sind C₁-C₃₀-Alkanole und -Cycloalkanole, C₂-C₁₀-Diole, C₁-C₂₀-Carbonsäuren, C₄-C₁₂-Carbonsäureanhydride sowie C₂-C₂₀-Dialkylether. Hierunter bevorzugt werden einwertige Alkanole mit 1 bis 20 Kohlenstoffatomen, insbesondere mit 1 bis 4 Kohlenstoffatomen, die gegebenenfalls zusammen mit den C₁-C₂₀-Dialkylethern eingesetzt werden können. Als Cokatalysator sind einwertige sekundäre C₃-C₂₀-Alkanole und tert-Butylether besonders bevorzugt. Beispielhaft seien genannt Isopropanol, 2-Butanol, sec-Pentanol, sec-Hexanol, sec-Heptanol, sec-Octanol und dergleichen. Besonders bevorzugt werden 2-Butanol, Isopropanol, Methyl-tert-butylether, Ethyl-tert-butylether verwendet.

Vorzugsweise beträgt das Molverhältnis von Bortrifluorid zu Cokatalysator 1:1 bis 1:10, insbesondere 1:1,1 bis 1:5 und besonders bevorzugt 1:1,2 bis 1:2,5. Das Bortrifluorid und der Cokatalysator können vorab unter Bildung eines Komplexes umgesetzt oder in situ im Reaktionsgemisch kombiniert werden.

Die Konzentration der Kombination von Bortrifluorid und Cokatalysator im Reaktor liegt in der Regel im Bereich von 0,005 bis 1 Gew.-%, bezogen auf die flüssige organische Phase, insbesondere im Bereich von 0,01 bis 0,7 Gew.-% und besonders bevorzugt im Bereich von 0,015 bis 0,5 Gew.-%.

Nach Erreichen des gewünschten Polymerisationsgrades wird der Katalysator abgetrennt und/oder deaktiviert und auf diese Weise die Polymerisation abgebrochen. Zur Katalysatordeaktivierung können Desaktivatoren, wie beispielsweise Wasser, Alkohole, Acetonitril, Ammoniak oder wässrige Lösungen von Mineralbasen oder Carbonaten, verwendet werden, die dem Reaktionsgemisch zugefügt werden. Hierzu können auch angesäuerte wässrige Lösungen verwendet werden. Anstatt den Katalysator im Reaktionsgemisch quantitativ zu deaktivieren, kann man ihn entweder quantitativ aus dem Reaktionsgemisch abtrennen oder teilweise aus dem Reaktionsgemisch abtrennen und den restlichen Katalysator im Reaktionsgemisch deaktivieren. Mit Vorteil erfolgt die Katalysatorabtrennung gemäß der Beschreibung der WO 99/31151.

Zur Abtrennung des Katalysators aus dem Reaktionsgemisch empfiehlt es sich, zuvor die Isobutenkonzentration auf weniger als 2 Gew.-%, vorzugsweise weniger als 1 Gew.-% und insbesondere weniger als 0,5 Gew.-%, bezogen auf das Reaktionsgemisch, zu verringern. Zur Abtrennung des Katalysators verwendet man bevorzugt lösliche Bortrifluorid-Komplex-Katalysatoren mit begrenzter Löslichkeit im Reaktionsgemisch und/oder kühlt das Reaktionsgemisch auf Temperaturen von beispielsweise 5 bis 30 Kelvin unterhalb der Reaktionstemperatur, vorzugsweise 10 bis 20 Kelvin unterhalb der Reaktionstemperatur, ab.

Im weiteren Gang der Aufarbeitung wird der Reaktionsaustrag zweckmäßigerweise einer oder mehreren Extraktionen - üblicherweise mit Wasser - zur Entfernung von Restmengen an Katalysator unterzogen.

Das Isobutenpolymerisat weist in der Regel ein zahlenmittleres Molekulargewicht Mₙ von 500 bis 50000 und einen Gehalt an endständigen Vinylidengruppen von mehr als 60 Mol-%, insbesondere mehr als 80 Mol-% auf. Die Dispersität M_{w}/Mₙ beträgt vorzugsweise nicht mehr als 1,9, insbesondere nicht mehr als 1,8.

Die vorliegende Erfindung wird durch die beigefügten Figuren und die nachfolgenden Beispiele und Vergleichsbeispiele näher veranschaulicht.

Fig. 1 zeigt den Längsschnitt eines Entspannungsbehälters, der mit Vorteil im erfindungsgemäßen Verfahren verwendet wird.

Fig. 2 zeigt einen Querschnitt durch den Entspannungsbehälter entlang der in Fig. 1 mit "A ----- A" gekennzeichneten Linie.

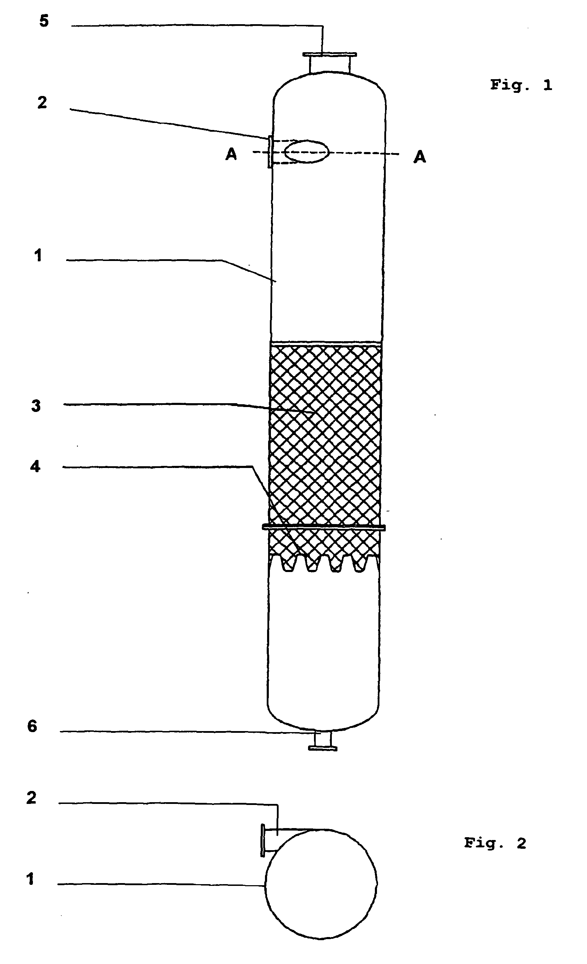

Der Entspannungsbehälter 1 gemäß Fig. 1 weist eine längliche zylindrische Form auf, wobei die Öffnung 2 zum Einführen des erwärmten Reaktionsaustrags tangential am Mantel angeflanscht ist. Über die Öffnung 5 kann die sich bei der Entspannung bildende Gasphase, über die Öffnung 6 die Flüssigphase abgezogen werden. Der Entspannungsbehälter 1 weist eine Füllkörperpackung 3 auf, die auf einem Träger 4 ruht. Fig. 2 zeigt die tangentiale Anordnung der Öffnung 2 bezüglich des zylindrischen Entspannungsbehälters 1 im Querschnitt.

### Beispiele 1 bis 3

Zur Herstellung eines Polyisobutens wurde gemäß der EP-A 628 575, Beispiel 1 verfahren: Der eingesetzte Isobuten-haltige Zulauf entsprach folgender Zusammensetzung:

| | |
|---|---|
| Isobutan | <1 Gew.-% |
| n-Butan | <1 Gew.-% |
| 1-Buten | <1 Gew.-% |
| trans-2-Buten | <1 Gew.-% |
| cis-2-Buten | <1 Gew.-% |
| Isobuten | 45 Gew.-% |
| Hexan | 54 Gew.-% |
| Butadien | <50 ppm |
| Wasser | etwa 2 ppm |

Im Verlauf von einer Stunde wurden 6000 g des obigen Zulaufs auf der Saugseite eines Schlaufenreaktors zugeführt, der mit einer integrierten Umwälzpumpe ausgestattet war, dessen Rohrdurchmesser 4 mm und dessen Volumen 1000 ml betrug. Es wurden Y mmol/1000 g Zulauf (siehe nachstehende Tabelle) Bortrifluorid zugegeben. Bezogen auf das Bortrifluorid wurde die 1,6-fache molare Menge 2-Butanol zugesetzt. Der Reaktor wurde so gekühlt, dass die Temperatur im Reaktionsmedium X °C (siehe nachstehende Tabelle) betrug. Die mittlere Verweilzeit des Reaktionsmediums im Reaktor lag bei 6,6 Minuten. Der Reaktionsaustrag wurde daraufhin mit 2000 g pro Stunde Wasser von 90 °C intensiv vermischt, wobei sich eine Temperatur zwischen 35 und 45 °C einstellte. Anschließend separierte man die beiden entstehenden Phasen. Die untere Phase enthielt das Waschwasser. Die obere Phase (im Folgenden Rohprodukt genannt) bestand aus Polyisobuten, Isobutenoligomeren, Restisobuten und dem Lösungsmittel Hexan und wurde in allen folgenden Beispielen verwendet.

Das Rohprodukt wurde in einem Drucksystem über einen mit 16 bar Dampf (etwa 200 °C) beheizten Wärmetauscher geleitet. Dabei stellte sich im Rohprodukt eine Temperatur von 140 °C und ein Druck von 14 bar ein. Anschließend wurde das erhitzte Rohprodukt tangential in das obere Drittel eines zylindrischen Entspannungsbehälters geleitet. Im mittleren Drittel des Entspannungsbehälters war eine Schüttschicht von Edelstahl-Pallringen angeordnet. Beim Einleiten in den Entspannungsbehälter wurde das erhitzte Rohprodukt von 14 auf 2,2 bar entspannt. Bei der Entspannung verdampften das Isobuten, Hexan und weitere leichtflüchtige Bestandteile des Rohprodukts. Sie wurden am Kopf des Entspannungsbehälters als Destillat abgezogen.

Die Ergebnisse sind in der nachfolgenden Tabelle zusammengestellt:

| Bsp. | Rohprodukt | X | Y |
|---|---|---|---|
| | i-Buten (%) | Reaktortemperatur [°C] | BF₃ [mmol/1000 g] |
| 1 | 5,5 | -17 | 7,1 |
| 2 | 1,3 | -17 | 19,8 |
| 3 | 0,9 | -8 | 19,8 |

| Bsp. | Destillationssumpf | | | Destillat | | |
|---|---|---|---|---|---|---|
| | Mₙ (PIB) | Vinyliden (%) | Dispersität | i-Octene (ppm) | t-BuOH (ppm) | t-Butylfluorid (ppm) |
| 1 | 2387 | 84,3 | 1,785 | 780 | 59 | <20 |
| 2 | 956 | 87,3 | 1,689 | 1359 | <20 | <20 |
| 3 | 658 | 85,3 | 1,652 | 1988 | <20 | <20 |

## Patentansprüche

1. Verfahren zur Aufarbeitung eines flüssigen Reaktionsaustrages der kationischen Polymerisation von Isobuten, der im Wesentlichen aus Polyisobuten, nicht umgesetzten Isobuten und einem inerten Verdünnungsmittel besteht, bei dem man den Reaktionsaustrag erwärmt und in einen Entspannungsbehälter entspannt, wobei sich der Reaktionsaustrag infolge der Entspannung in eine das Polyisobuten enthaltende Flüssigphase und eine wenigstens 90 % des im Reaktionsaustrag enthaltenen Isobutens und wenigstens 30 % des im Reaktionsaustrag enthaltenen Verdünnungsmittels enthaltende Gasphase auftrennt.

2. Verfahren nach Anspruch 1, wobei die Druckdifferenz der Entspannung wenigstens 1 bar beträgt.

3. Verfahren nach einem der vorhergehenden Ansprüche, wobei man einen Entspannungsbehälter mit im Wesentlichen kreisförmigen Querschnitt verwendet und den erwärmten Reaktionsaustrag tangential einführt.

4. verfahren nach einem der vorhergehenden Ansprüche, wobei man den erwärmten Reaktionsaustrag in den mittleren bis oberen Bereich des Entspannungsbehälters einführt und die sich bildende Flüssigphase über trennwirksame Einbauten im unteren Bereich des Entspannungsbehälters leitet.

5. Verfahren nach Anspruch 4, wobei man als trennwirksame Einbauten eine Füllkörperpackung verwendet.

6. Verfahren nach einem der vorhergehenden Ansprüche, wobei man den Reaktionsaustrag auf eine Temperatur von 40 bis 200 °C erwärmt.

7. Verfahren nach einem der vorhergehenden Ansprüche, wobei man die Gasphase zu einem flüssigen Isobuten-Verdünnungsmittelgemisch kondensiert, das in die Polymerisationsreaktion zurückgeführt wird.

## Claims

1. A process for working up a liquid reaction discharge of the cationic polymerization of isobutene, which substantially comprises polyisobutene, unconverted isobutene and an inert diluent, in which the reaction discharge is heated and is let down into a flash container, the reaction discharge separating, as a result of the flashing, into a polyisobutene-comprising liquid phase and a gas phase comprising at least 90% of the isobutene present in the reaction discharge and at least 30% of the diluent present in the reaction discharge.

2. The process according to claim 1, wherein the pressure difference in the flash is at least 1 bar.

3. The process according to any of the preceding claims, wherein a flash container having a substantially circular cross section is used and the heated reaction discharge is introduced tangentially.

4. The process according to any of the preceding claims, wherein the heated reaction discharge is introduced into the middle to upper region of the flash container and the resulting liquid phase is passed over internals having separation activity and present in the lower region of the flash container.

5. The process according to claim 4, wherein a packing is used as the internals having separation activity.

6. The process according to any of the preceding claims, wherein the reaction discharge is heated to a temperature of from 40 to 200°C.

7. The process according to any of the preceding claims, wherein the gas phase is condensed to a liquid isobutene/diluent mixture which is recycled to the polymerization reaction.

## Revendications

1. Procédé pour le traitement final d'un courant liquide de décharge de réaction de la polymérisation cationique d'isobutène, qui est essentiellement constitué de polyisobutène, d'isobutène n'ayant pas réagi et d'un diluant inerte, dans lequel on chauffe le courant de décharge de réaction et on le détend dans un récipient de détente, le courant de décharge de réaction se fractionnant, par suite de la détente, en une phase liquide contenant le polyisobutène et une phase gazeuse contenant au moins 90 % de l'isobutène contenu dans le courant de décharge de réaction et au moins 30 % du diluant contenu dans le courant de décharge de réaction.

2. Procédé selon la revendication 1, dans lequel la différence de pression de la détente est d'au moins 1 bar.

3. Procédé selon l'une quelconque des revendications précédentes, dans lequel on utilise un récipient de détente à section transversale essentiellement circulaire et on introduit tangentiellement le courant chauffé de décharge de réaction.

4. Procédé selon l'une quelconque des revendications précédentes, dans lequel on introduit le courant chauffé de décharge de réaction dans la zone médiane à supérieure du récipient de détente et on conduit la phase liquide qui se forme, en la faisant passer sur des inserts à effet séparateur, dans la zone inférieure du récipient de détente.

5. Procédé selon la revendication 4, dans lequel on utilise comme inserts à effet séparateur un garnissage de corps de remplissage.

6. Procédé selon l'une quelconque des revendications précédentes, dans lequel on chauffe à une température de 40 à 200°C le courant de décharge de réaction.

7. Procédé selon l'une quelconque des revendications précédentes, dans lequel on condense la phase gazeuse en un mélange liquide d'isobutène-diluant, qui est renvoyé dans la réaction de polymérisation.
